# FASCICULE DE BREVET EUROPEEN

(11) **EP 0 838 235 B1**
(45) Date de publication et mention de la délivrance du brevet: **02.01.2004**
(21) Numéro de dépôt: 97402517.3
(22) Date de dépôt: 23.10.1997
(51) Int. Cl.: A61N 1/39, A61N 1/368

(54) **Dispositif médical actif du type défibrillateur/cardioverteur implantable à discrimination perfectionnée des tachycardies**
Aktive medizinische Vorrichtung von der Art eines Defibrillator/Kardiovertierer mit verbesserter Unterscheidung von Tachykardien
Defibrillator/cardioverter type active medical device with improved discrimination of tachycardia

(30) Priorité: 25.10.1996 FR 9613083
(43) Date de publication de la demande: 29.04.1998
(73) Titulaire: ELA MEDICAL S.A., 92541 Montrouge (FR)
(72) Inventeur: Nitzsche, Rémi, 78950 Gambais (FR); Iscolo, Nicolas, 78120 Saint Cyr L'Ecole (FR); Henry, Christine, 75014 Paris (FR); Bonnet, Jean-Luc, 92150 Montrouge (FR); Limousin, Marcel, 92120 Montrouge (FR); Kroiss, Daniel, 67590 Schweighouse (FR)
(74) Mandataire: Dupuis-Latour, Dominique

(56) Documents cités:
- EP-A- 0 550 344
- EP-A- 0 617 980
- EP-A- 0 626 182
- WO-A-92/09331
- WO-A-93/02746

## Description

La présente invention concerne les dispositifs médicaux implantables actifs (au sens de la directive 90/385/CEE du 20 juin 1990 du Conseil des communautés européennes), et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie. Ces modes de thérapie incluent également un mode de stimulation programmée à haute fréquence ou "ATP" (AntiTachycardic Pacing).

Ces dispositifs sont communément appelés "défibrillateurs implantables" ou "appareils de cardioversion" (étant entendu que l'invention couvre aussi bien les défibrillateurs/cardioverteurs/stimulateurs implantables que les défibrillateurs/stimulateurs implantables).

Ces dispositifs comprennent un générateur d'impulsions chargé de surveiller l'activité cardiaque et de générer des impulsions de stimulation ou de haute énergie quand le coeur présente une arythmie ventriculaire susceptible d'être traitée (quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation").

Ce choc doit être délivré lorsque l'on détecte une tachycardie ventriculaire (TV), mais à condition qu'il s'agisse bien d'une véritable TV, et non d'une tachycardie supra-ventriculaire (TSV) ; en effet, dans ce dernier cas, la tachycardie est d'origine auriculaire et le choc qui serait délivré serait sans effet puisque l'électrode de défibrillation ou, le cas échéant, de stimulation, ne se trouve pas dans cette région.

La tachyarythmie correspond à un rythme cardiaque rapide anormal et recouvre la fibrillation ventriculaire (FV), la tachycardie ventriculaire (TV), la tachycardie sinusale (TS) et la tachycardie supra-ventriculaire (TSV). La tachycardie supra-ventriculaire recouvre la tachycardie auriculaire, le *flutter* auriculaire et la fibrillation auriculaire.

Le diagnostic des tachycardies est opéré, de manière en elle-même connue (voir notamment le EP-A-0 626182 au nom de ELA Médical), à partir de critères tels que la fréquence ventriculaire, la stabilité des intervalles ventriculaires (intervalles RR), l'analyse de l'association auriculo-ventriculaire (révélée par la stabilité de l'intervalle PR) et le mode de démarrage des tachycardies (présence d'une accélération brusque et cavité d'origine, ventriculaire ou auriculaire).

Plus précisément, l'appareil décrit dans le brevet précité définit qu'il y a stabilité des intervalles RR lorsque le pic d'autocorrélation, divisé par le total d'autocorrélation, excède un rapport déterminé (le pic d'autocorrélation est le nombre maximal d'intervalles récents dans le ventricule qui satisfont à un critère de stabilité prédéterminé).

Il définit qu'il y a stabilité de conduction lorsque la valeur du pic d'intercorrélation, divisée soit par la valeur du pic d'autocorrélation, soit, en variante, par le total d'intercorrélation, excède un rapport déterminé (le pic d'intercorrélation est le nombre maximal d'intervalles de conduction en provenance de l'oreillette qui satisfont un critère de stabilité prédéterminé). En d'autres termes, dans le premier cas précité on compare la stabilité de la conduction entre les deux chambres à celles des intervalles dans le ventricule, tandis que dans le second cas on exprime la stabilité de la conduction entre les deux chambres en fonction de la totalité des conductions présumées.

Lorsqu'il n'y a pas de stabilité dans le ventricule (intervalles RR instables), cela signifie que la tachyarythmie n'est probablement pas susceptible d'être interrompue par une thérapie appliquée au ventricule, et le procédé définit qu'elle n'est pas susceptible d'être interrompue dans cette chambre.

Inversement, lorsqu'il y a stabilité dans le ventricule (intervalles RR stables), mais pas de stabilité de conduction (absence d'association auriculo-ventriculaire), cela signifie que la tachyarythmie a probablement son origine dans le ventricule, et le procédé définit qu'elle est susceptible d'être interrompue par une thérapie appliquée dans cette chambre.

Toujours lorsqu'il y a stabilité dans le ventricule (RR stables), mais qu'il y a également stabilité de conduction (association auriculo-ventriculaire avérée), le procédé en question détermine si la conduction en provenance de la seconde chambre est en 1:1 ou n:1, en comparant le pic d'intercorrélation au total d'intercorrélation. Dans le cas d'une association 1:1 on considère un autre critère, à savoir la présence ou non d'une accélération (accélération brusque) prenant naissance avec une dissociation, pour déterminer si la tachyarythmie est susceptible ou non d'être interrompue par stimulation et/ou par choc électrique.

En revanche, dans le cas d'une association en n:1, on conclut que la tachyarythmie n'est en aucune façon susceptible d'être interrompue par stimulation du ventricule, ceci parce qu'elle a son origine dans l'oreillette.

On observe cependant dans certains cas cliniques des défaillances de l'algorithme de classification.

La demande française 96 07533 décrit à cet égard un perfectionnement qui permet de détecter la présence de cycles longs afin d'assurer une discrimination supplémentaire entre TV et TSV, notamment en présence d'une FA conduite et installée présentant des intervalles RR réguliers pendant une durée suffisante pour conduire à un faux diagnostic de TV (rythme régulier, dissocié entre oreillette et ventricule) mais l'observation clinique révèle que, dans certains cas de tachyarythmies nécessitant réellement une thérapie, cette thérapie n'a pas été appliquée, ou l'a été trop tardivement.

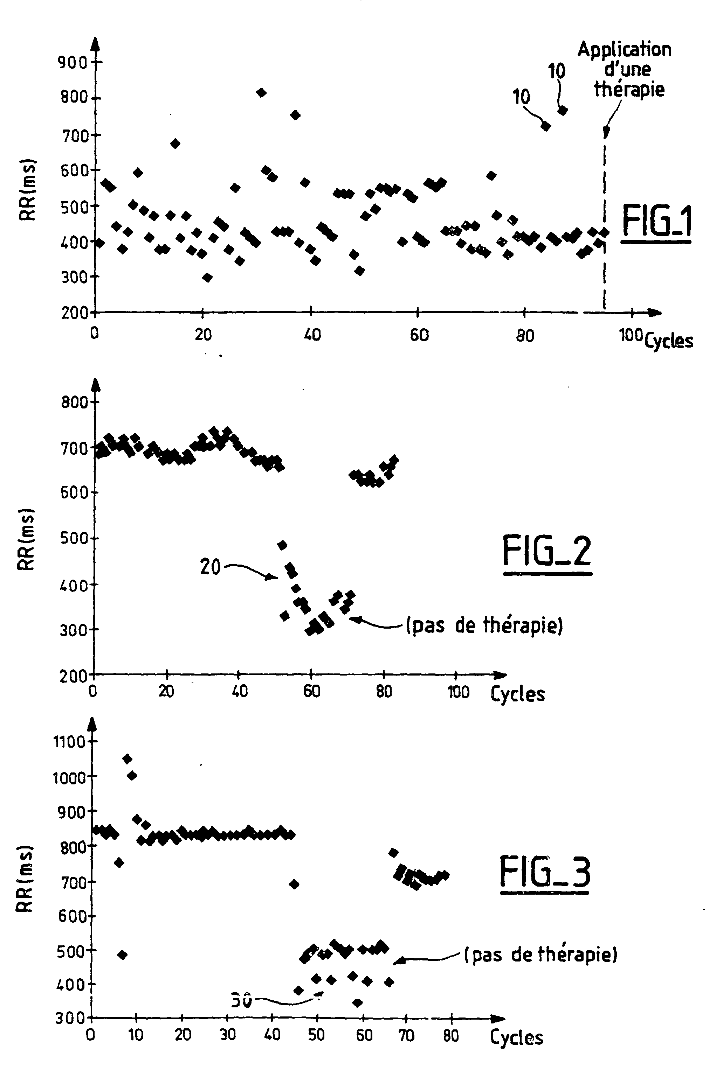

On va expliquer ces cas particuliers en référence aux figures 1 à 3, qui représentent l'évolution de la valeur de l'intervalle RR (un intervalle court correspondant à un rythme ventriculaire élevé) au cours d'une succession de cycles.

Dans le cas illustré figure 1, les intervalles RR deviennent rapidement très stables, avec une différence typique de 63 ms entre la valeur minimale et la valeur maximale sur les dix-sept cycles précédant la thérapie, appliquée au cycle n° 95 : l'algorithme a en effet interprété cette situation comme correspondant à une stabilité des intervalles RR avec absence d'association, conduisant à un diagnostic de TV, normalement susceptible d'une thérapie.

Néanmoins, la présence de cycles longs, en 10, indique de façon claire au clinicien qu'il ne s'agit pas d'une TV, mais d'une TSV non redevable d'un choc.

On voit ainsi que, dans cet exemple, l'appareil a produit un faux diagnostic positif (indication d'une TV alors qu'il s'agissait d'une TSV).

Dans le cas des figures 2 et 3, inversement, une variation progressive de RR (en 20 sur la figure 2), ou une instabilité de RR sur des cycles très courts (en 30 sur la figure 3), a été interprétée par l'appareil comme une instabilité du rythme RR, concluant ainsi ― à tort ― qu'il n'y avait pas lieu d'appliquer une thérapie (dans ces exemples, le critère pour diagnostiquer et traiter une TV était la détection d'au moins six cycles dans le pic d'autocorrélation (définissant la stabilité des intervalles RR), ceci durant huit cycles au moins en continu ― alors que, dans le cas des figures 2 et 3, la durée de persistance de la stabilité n'avait pas dépassée cinq et six cycles, respectivement.

Dans ces deux autres exemples, on a donc abouti à un faux diagnostic négatif, c'est-à-dire que l'on a cru à une TSV alors qu'il s'agissait d'une TV.

Le but de l'invention est de remédier aux inconvénients précités, en proposant de perfectionner les appareils existant afin d'éliminer tout risque de faux diagnostic de TV (faux positif ou faux négatif) dans certaines situations atypiques, c'est-à-dire d'accroître la spécificité et la fiabilité de l'analyse des tachyarythmies.

Le dispositif médical de l'invention est un défibrillateur ou cardioverteur d'un type en lui-même connu, tel que décrit par exemple dans le EP-A-0 626 182 précité, c'est-à-dire qui comprend : des moyens de délivrance d'une thérapie de défibrillation et/ou de cardioversion et/ou de stimulation antitachycardique ventriculaire et/ou auriculaire ; des moyens de recueil de l'activité ventriculaire et auriculaire ; des moyens pour suspecter et confirmer la présence d'épisodes de tachycardie dans l'activité ainsi recueillie, ces moyens comprenant des moyens pour analyser pendant une période d'analyse la stabilité des intervalles RR et la stabilité des intervalles PR associés ; des moyens classificateurs, propres à discriminer les tachycardies ainsi détectées entre tachycardies ventriculaires et tachycardies supra-ventriculaires ; et des moyens pour, sélectivement, (i) commander les moyens de délivrance de la thérapie en présence de tachycardies ventriculaires ou (ii) inhiber ces mêmes moyens en présence de tachycardies supra-ventriculaires ou déclencher une thérapie auriculaire.

Les moyens discriminateurs comprennent des moyens propres à déterminer la présence ou l'absence d'une accélération brusque du rythme des intervalles RR, et pour déterminer le cas échéant l'origine ventriculaire ou atriale d'une telle accélération. Selon l'invention, ces moyens opèrent dans le cas de la détection d'intervalles RR instables.

De préférence, les moyens propres à déterminer la présence ou l'absence d'une accélération brusque opèrent dans le cas de la détection d'intervalles RR instables avec absence d'intervalles RR longs détectés dans la période d'analyse.

Très avantageusement, les moyens classificateurs comprennent en outre des moyens propres à déterminer la présence ou l'absence d'intervalles RR longs dans une période d'analyse donnée, en cas de détection d'intervalles RR stables avec intervalles PR instables.

D'autres caractéristiques de l'invention apparaîtront à la lecture de la description détaillée ci-dessous d'un exemple de mise en oeuvre.

Les figures 1 à 3, précitées, sont des chronogrammes décrivant des relevés cliniques de variations du rythme RR au cours d'un certain nombre de cycles ayant entraîné des faux diagnostics positifs ou négatifs.

La figure 4 est un arbre de décision expliquant la manière dont sont opérées la discrimination et la classification des tachyarythmies selon l'invention.

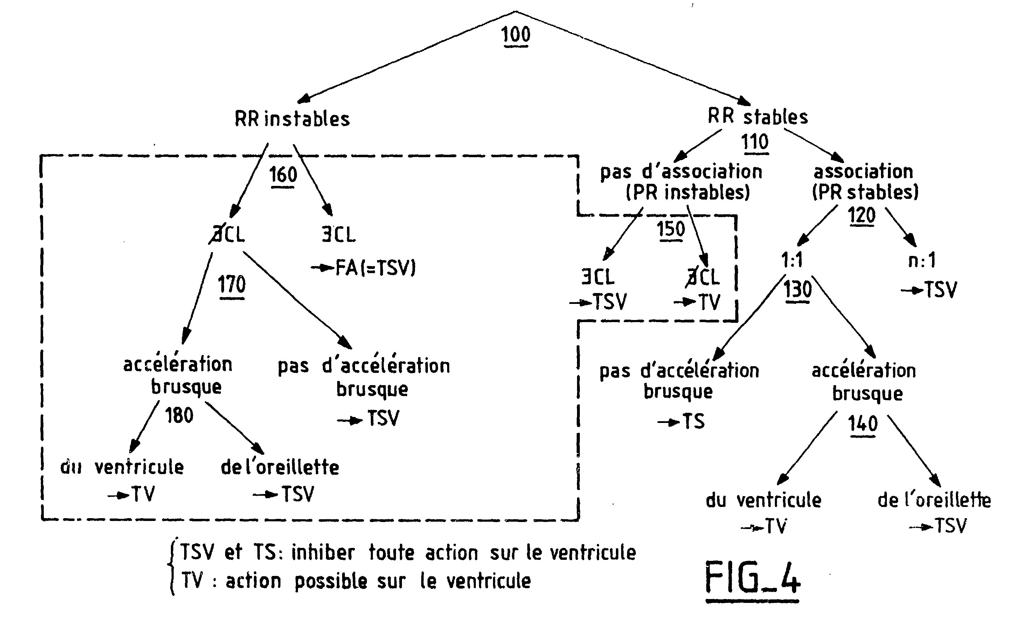

Sur la figure 4, on a représenté de façon schématique l'arbre de décision de l'algorithme de détection et de classification des tachyarythmies d'un défibrillateur tel que le modèle Defender 9001 de ELA Médical, algorithme que l'on a perfectionné selon les enseignements de la présente invention.

Plus précisément, la partie de l'arbre de décision enfermée dans un cadre en tiretés correspond au perfectionnement de l'invention, tandis que le reste de l'arbre de décision correspond aux éléments de l'algorithme déjà connus et décrits notamment par le EP-A-0 626182 précité, auquel on pourra se reporter pour de plus amples détails.

La première étape de l'analyse (test 100) consiste à déterminer si on a ou non une stabilité du rythme RR.

Si tel est le cas, on procède ensuite à l'examen (étape 110) de la présence ou non d'une association auriculo-ventriculaire.

Dans l'affirmative, on examine alors (test 120) s'il s'agit d'une association en 1:1 ou en n:1. Dans ce dernier cas, on considère qu'il y a TSV, on inhibe toute action sur le ventricule et on déclenche éventuellement une action sur l'oreillette.

Dans le cas d'une association 1:1, on recherche (test 130) s'il y a eu ou non accélération brusque du rythme. Le critère d'analyse de l'accélération du rythme ventriculaire et la détermination de l'origine de cette accélération sont décrits dans le EP-A-0 626 182 précité.

S'il y a eu peu ou pas d'accélération, on considère que l'on est en présence d'une tachycardie sinusale, vraisemblablement d'origine physiologique, et l'on inhibe toute action sur le ventricule.

Si, en revanche, on a détecté une accélération brusque des intervalles RR, on recherche (test 140) l'origine de cette accélération : ventriculaire (il s'agit alors d'une TV et on autorise une action sur le ventricule) ou auriculaire (il s'agit d'une TSV, on inhibe toute action sur le ventricule et on déclenche éventuellement une action sur l'oreillette).

La partie de l'arbre de décision que l'on vient de décrire est mise en oeuvre par l'algorithme de l'art antérieur.

Dans le cas où, au test 100, on détectait un rythme RR instable, l'algorithme de l'art antérieur considérait systématiquement qu'il y avait TSV et inhibait toute action sur le ventricule.

Il en était de même dans le cas où, au test 110, on détectait une absence d'association en présence d'intervalles RR stables.

Comme on l'a expliqué au début de la présente description, ceci pouvait conduire à des faux diagnostics dans certaines situations cliniques atypiques.

Pour y remédier, l'invention propose, dans l'un et l'autre de ces cas, d'examiner la présence éventuelle de cycles RR longs afin d'améliorer la classification des tachyarythmies.

Ainsi, dans le cas où, au test 110, on avait détecté une absence d'association, on procède à une discrimination supplémentaire (test 150), pour détecter la présence ou l'absence de cycles longs.

Le critère "présence d'un cycle long" est défini comme étant vérifié si l'on détecte au moins un cycle dont la durée de l'intervalle RR est supérieure à une valeur prédéfinie. Il peut s'agir notamment d'une durée que l'on peut exprimer sous la forme RRmax + StabRR, RRmax étant la limite supérieure du pic d'autocorrélation (définissant la stabilité des intervalles RR), et StabRR étant une valeur paramétrée définissant un intervalle de garde entre cette limite supérieure et la plage de détection des intervalles considérés comme longs.

S'il y a présence de cycle long, on considère qu'il y a TSV, on inhibe toute action sur le ventricule et on déclenche éventuellement une action sur l'oreillette.

Ainsi, dans l'exemple de la figure 1, on interdit l'application d'une thérapie après la survenue des cycles longs 10, évitant ainsi un faux positif.

Inversement, si l'on ne détecte pas de cycle long, on considère qu'il y a TV et l'on autorise une action sur le ventricule.

Si, au test 100, on détecte une instabilité du rythme RR, on procède à une discrimination supplémentaire (test 160) pour rechercher, comme on l'avait fait au test 150, la présence ou l'absence d'un cycle long.

La présence de cycle long révèle une fibrillation auriculaire (FA), c'est-à-dire une fréquence anormalement élevée du rythme auriculaire conduit au ventricule. Il s'agit d'une forme particulière de TSV, qui implique d'inhiber toute action sur le ventricule, puisque celle-ci serait sans effet, ou pourrait même être néfaste au patient.

Dans le cas où, en revanche, l'on ne détecte pas de cycle long, on recherche (test 170) s'il y a eu ou non accélération brusque du rythme RR, de la même façon qu'au test 130.

L'absence d'accélération brusque révèle qu'il s'agit d'une TSV, et l'on doit donc inhiber toute action sur le ventricule.

En présence d'une accélération brusque, on recherche son origine (test 180, opéré de la même manière que le test 140).

Si l'accélération provient de l'oreillette, il s'agit d'une TSV et on doit inhiber toute action sur le ventricule.

En revanche, si cette accélération trouve son origine dans le ventricule, il s'agit bien d'une TV, et l'on doit permettre une action sur le ventricule.

On remédie ainsi aux faux négatifs des exemples des figures 2 et 3, en permettant la délivrance d'une thérapie malgré l'instabilité du rythme RR en 20 et 30 respectivement.

Dans une variante simplifiée de l'invention, on peut omettre le test référencé 160, c'est-à-dire que, en cas d'intervalles RR instables avérés, on procède directement au test d'accélération (test 170), sans rechercher si, dans ce cas, il y a ou non présence d'un cycle long.

## Revendications

1. Dispositif médical actif du type défibrillateur/cardioverteur implantable, comprenant :
― des moyens de délivrance d'une thérapie de défibrillation et/ou de cardioversion et/ou de stimulation antitachycardique ventriculaire et/ou auriculaire,
― des moyens de recueil de l'activité ventriculaire et auriculaire,
― des moyens pour suspecter et confirmer la présence d'épisodes de tachycardie dans l'activité ainsi recueillie, ces moyens comprenant des moyens pour analyser pendant une période d'analyse la stabilité des intervalles RR et la stabilité des intervalles PR associés,
― des moyens classificateurs (110-140), propres à discriminer les tachycardies ainsi détectées entre tachycardies ventriculaires et tachycardies supra-ventriculaires, ces dits moyens classificateurs comprenant en outre des moyens (170) propres à déterminer la présence ou l'absence d'une accélération brusque du rythme des intervalles RR, et à déterminer le cas échéant l'origine ventriculaire ou atriale d'une telle accélération, et
― des moyens pour, sélectivement, (i) commander les moyens de délivrance de la thérapie en présence de tachycardies ventriculaires ou (ii) inhiber ces mêmes moyens en présence de tachycardies supra-ventriculaires ou déclencher une thérapie auriculaire,
dispositif **caractérisé en ce que** lesdits moyens (170) propres à déterminer la présence ou l'absence d'une accélération brusque du rythme des intervalles RR, et à déterminer le cas échéant l'origine ventriculaire ou atriale d'une telle accélération, opèrent dans le cas de la détection d'intervalles RR instables.

2. Le dispositif médical de la revendication 1, dans lequel les moyens (160, 170) propres à déterminer la présence ou l'absence d'une accélération brusque opèrent dans le cas de la détection d'intervalles RR instables avec absence d'intervalles RR longs détectés dans la période d'analyse.

3. Le dispositif médical de la revendication 1, dans lequel les moyens classificateurs comprennent en outre des moyens (150) propres à déterminer la présence ou l'absence d'intervalles RR longs dans une période d'analyse donnée, en cas de détection d'intervalles RR stables avec intervalles PR instables.

## Claims

1. An active medical device of the defibrillator/cardioverter type, comprising:
- means for delivering a defibrillation and/or cardioversion and/or ventricular and/or atrial antitachycardic stimulation therapy;
- means for collecting the atrial and ventricular activity;
- means for suspecting and confirming the presence of tachycardia episodes in the collected activity, said means comprising means for analyzing during an analysis period the stability of the RR intervals and the stability of the associated PR intervals;
- classification means (110-140), able to discriminate tachycardias detected in this manner between ventricular tachycardias and supra-ventricular tachycardias, said classification means further comprising means (170) for determining the presence or the absence of a rapid acceleration of the rhythm of the RR intervals, and for determining, if necessary, the atrial or ventricular origin of such acceleration, and
- means for, selectively, (i) controlling the means for delivering the therapy in the presence of ventricular tachycardias or (ii) inhibiting these means for delivering the therapy presence of a supra-ventricular tachycardias or triggering an atrial therapy,
said device being **characterized in that** said means (170) for determining the presence or the absence of a rapid acceleration of the rhythm of the RR intervals operate in the case of the detection of unstable RR intervals.

2. The medical device of claim 1, in which the means (160, 170) for determining the presence or the absence of a rapid acceleration operate in the case of the detection of unstable RR intervals with an absence of long RR intervals detected during the period of analysis.

3. The medical device of claim 1, in which the classification means further comprises means (150) for determining the presence or the absence of long RR intervals in a given period of analysis, in the case of detection of stable RR intervals with unstable PR intervals.

## Patentansprüche

1. Aktive medizinische Vorrichtung vom Typ implantierbarer Defibrillator/Kardiovertierer, umfassend:
Mittel zum Verabreichen einer Therapie der Defibrillation und/oder der Kardioversion und/oder der ventrikulären und/oder aurikulären antitachykardischen Stimulation,
Mittel zum Empfangen der ventrikulären oder aurikulären Aktivität,
Mittel zum Vermuten und Bestätigen des Vorhandenseins von Episoden der Tachykardie in der so empfangenen Aktivität, wobei diese Mittel zum Analysieren der Stabilität der RR-Intervalle und der Stabilität der verbundenen PR-Intervalle aufweisen,
Mittel zum Klassifizieren (110-140), geeignet, um unter den so gefundenen Tachykardien zwischen ventrikulären und supraventrikulären Tachykardien zu unterscheiden, wobei die Mittel zur Klassifikation ferner Mittel (170) umfassen, die geeignet sind, das Vorhandensein oder die Abwesenheit einer plötzlichen Beschleunigung des Rhythmus der RR-Intervalle zu bestimmen und gegebenenfalls den ventrikulären oder atrialen Ursprung einer derartigen Beschleunigung zu bestimmen, und
Mittel zum selektiven (i) Steuern der Mittel zur Verabreichung der Therapie bei Vorhandensein von ventrikulären Tachykardien oder (ii) Sperren derselben Mittel bei Vorhandensein von supraventrikulären Tachykardien oder Auslösen einer aurikulären Therapie,
wobei die Vorrichtung **dadurch gekennzeichnet ist, dass**
die Mittel (170), die geeignet sind, das Vorhandensein oder die Abwesenheit einer plötzlichen Beschleunigung des Rhythmus der RR-Intervalle zu bestimmen und gegebenenfalls den ventrikulären oder atrialen Ursprung einer derartigen Beschleunigung zu bestimmen, im Fall des Findens von instabilen RR-Intervallen arbeiten.

2. Medizinische Vorrichtung gemäß Anspruch 1, in welcher die Mittel (160, 170), die geeignet sind, das Vorhandensein oder die Abwesenheit einer plötzlichen Beschleunigung zu bestimmen, im Fall des Findens von instabilen RR-Intervallen bei Abwesenheit von langen RR-Intervallen, die in der Analyse-Periode festgestellt wurden, arbeiten.

3. Medizinische Vorrichtung gemäß Anspruch 1, in welcher die Mittel zur Klassifikation ferner Mittel (150) umfassen, die geeignet sind, das Vorhandensein oder die Abwesenheit von langen RR-Intervallen in einer gegebenen Analyse-Periode zu bestimmen, im Fall des Findens von stabilen RR-Intervallen mit instabilen PR-Intervallen.
